# EUROPÄISCHE PATENTSCHRIFT

(11) **EP 1 242 405 B1**
(45) Veröffentlichungstag und Bekanntmachung des Hinweises auf die Patenterteilung: **18.08.2004**
(21) Anmeldenummer: 00983354.2
(22) Anmeldetag: 20.12.2000
(51) Int. Cl.: C07D 401/06

(54) **VERFAHREN ZUR HERSTELLUNG VON 4-(HETEROARYL-METHYL)-1(2H)-PHTHALAZINONEN**
METHOD FOR PRODUCING 4-(HETEROARYL-METHYL)-1(2H)-PHTHALAZINONES
PROCEDE DE PREPARATION DE 4-(HETEROARYLE-METHYLE)-1(2H)-PHTALAZINONES

(30) Priorität: 23.12.1999 DE 19963607
(43) Veröffentlichungstag der Anmeldung: 25.09.2002
(73) Patentinhaber: Schering Aktiengesellschaft, 13353 Berlin (DE)
(72) Erfinder: PETROV, Orlin, 14199 Berlin (DE); HEINER, Thomas, 10179 Berlin (DE); NEH, Harribert, 12247 Berlin (DE); KRÜGER, Martin, 13465 Berlin (DE)
(86) Internationale Anmeldenummer: PCT/EP2000/013027
(87) Internationale Veröffentlichungsnummer: WO 2001/047912

(56) Entgegenhaltungen:
- EP-A- 0 634 404
- EP-A- 0 722 936
- WO-A-00/05218
- WO-A-00/05219
- WO-A-98/27066
- WO-A-98/35958
- WO-A-99/32456

## Beschreibung

Die Erfindung betrifft ein Verfahren zur Herstellung von 4-(Heteroaryl-methyl)-1(2H)-phthalazinonen.

4-(Heteroaryl-methyl)-1(2H)-phthalazinon, insbesondere das 4-(4-Pyridylmethyl)-1(2H)-phthalazinon, sind wertvolle Zwischenprodukte bei der Herstellung von Phthalazinderivaten, die sich durch pharmakologisch interessante Eigenschaften wie z. B. Hemmung der Angiogenese (WO 98/35958), Inhibierung der cGMP Phosphodiesterase (EP 0 722 936), entzündungshemmende und blutdrucksenkende Wirkung (DE OS 2 021 195) auszeichnen und damit neue therapeutische Möglichkeiten, insbesondere zur Behandlung von Krebs und Herzkrankheiten eröffnen.

Die Herstellung von beispielsweise 4-(4-Pyridylmethyl)-1(2H)-phthalazinon erfolgt nach der bisher bekannten Methode durch die Reaktion von Phthalsäureanhydrid und 4-Methylpyridin bei ca. 200 ° C und anschließende Umsetzung des erhaltenen Kondensationsproduktes (γ-Pyrophthalon) mit Hydrazin-Überschuß bei 130 ° C (DE AS 1 061 788). Nachteile dieser Methode sind die geringe Ausbeute (< 50%), niedrige Produktqualität und vor allem die erforderliche sehr hohe Temperatur der Kondensationsreaktion, die eine großtechnische Anwendung der Methode sehr erschwert.

Alternativ kann 4-(4-Pyridylmethyl)-1(2H)-phthalazinon durch Kondensation von Phthalid mit 4-Pyridinaldehyd in Gegenwart von Natriummethylat und anschließende Reaktion des erhaltenen 2-(4(*1H*)-Pyridinyliden)-4,5,6,7-tetrahydroinden-1,3-dions mit großem Überschuß (16 Eq.) Hydrazin bei 130 ° C (WO 98/35958) hergestellt werden. Die Ausbeute über die 2 Stufen beträgt ca. 40 % der Theorie. Sehr problematisch bei dieser Methode ist die Handhabung des großen Überschusses des karzinogenen Hydrazins bei einer Temperatur, die oberhalb der Zersetzungstemperatur von Hydrazin (ca. 120 ° C) liegt. Dabei ist es kaum möglich, bei der Aufarbeitung und Isolierung des Produktes den sehr niedrigen Grenzwert für Hydrazin in der Luft (MAK 0,008 ppm) oder in den Abwässern einzuhalten.

Aus der WO 99/32456 ist eine Umsetzung bekannt, die jedoch mit etwa 100fachem Überschuß an Hydrazin und bei einer Reaktionstemperatur in der Nähe der Zersetzungstemperatur von Hydrazin (ca. 120°C) durchgeführt wird. In technischem Maßstab ist ein solches Verfahren sehr problematisch. Ferner ist die Ausbeute vergleichsweise gering.

Es wäre daher wünschenswert, ein durchführbares Verfahren zur Herstellung von 4-(Heteroaryl-methyl)-1(2H)-phthalazinonen, speziell von 4-(4-Pyridylmethyl)-1(2H)-phthalazinon zu haben, das die technischen (Reaktion bei 200°C), Sicherheits(Erhitzen von Hydrazin auf 130°C) und Umweltprobleme (großer Überschuß an Hydrazin) der bekannten Verfahren vermeidet.

Die bekannten Nachteile werden nun durch das erfindungsgemäße Verfahren überwunden.

Gegenstand der Erfindung ist somit ein Verfahren zur Herstellung von 4-(Heteroarylmethyl)-halogen-1(2H)-phthalazinonen der allgemeinen Formel I 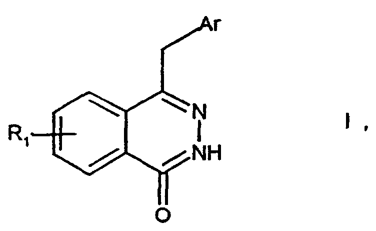 worin R¹ = Fluor, Chlor, Brom oder Wasserstoff und
Ar = Pyridin, Pyrazin oder Pyrimidin bedeutet, dadurch gekennzeichnet, daß substituierte Phthalidyl-3-triphenylphosphonium-Salze der allgemeinen Formel II 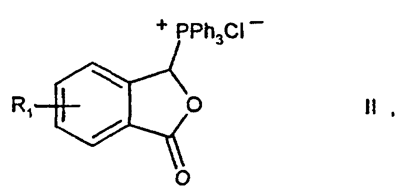 worin R¹ = Fluor, Chlor, Brom oder Wasserstoff bedeutet,
mit Aldehyden der allgemeinen Formel III

Ar-CHO III,

worin Ar = Pyridin, Pyrazin oder Pyrimidin bedeutet, in Gegenwart einer Base und anschließender Reaktion mit Hydrazinhydrat, und gegebenenfalls unter sauren Bedingungen, umgesetzt werden.

Der Rest R¹, sollte dieser für Halogen stehen, kann sich in jeder beliebigen Position am Phenylring innerhalb des Pynazinonsystems befinden, also in der 1, 2, 3 oder 4 Position. Als geeignete Ar-Reste sind Pyridin, Pyrimidin oder Pyrazin zu nennen. Geeignete Aldehyde sind beispielsweise 2-, 3- oder 4-Pyridin-aldehyd, 2-Methyl-4-pyridin-aldehyd, 3-Methyl-4-pyridin-aldehyd, 4-Pyrimidin-aldehyd, 5- Pyrimidinaldehyd, 3-Pyrazin-aldehyd oder 4-Pyrazin-aldehyd.

Also beispielsweise durch die Umsetzung von Phthalidyl-3-triphenylphosphonium-Salz mit 4-Pyridinaldehyd in Gegenwart einer Base (basischer Hilfsstoff), anschließende Reaktion mit Hydrazinhydrat und saure Behandlung des Reaktionsgemisches. Im speziellen gelöst durch die Umsetzung in einem Lösungsmittel von Phthalidyl-3-triphenylphosphonium-Salzen mit 4-Pyridinaldehyd in Gegenwart einer Base (basischer Hilfsstoff), anschließende Reaktion mit 1-1.1 Eq. Hydrazinhydrat und anschließende Behandlung des Reaktionsgemisches mit 0,1-0,3 Eq.
Essigsäureanhydrid.

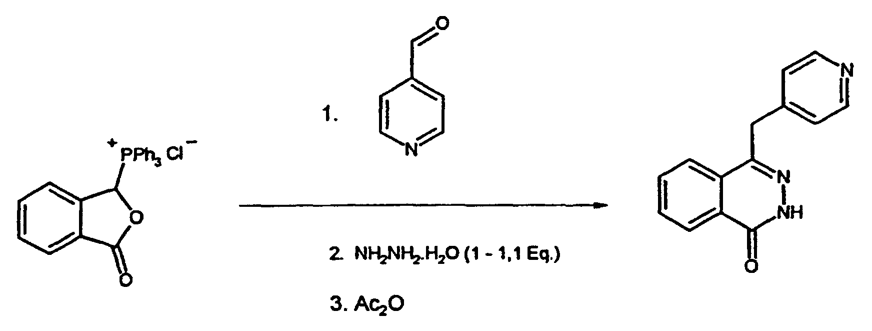

Zur Isolierung des Produktes wird das Reaktionsgemisch mit einer wässerigen Säure versetzt, das Lösungsmittel abdestilliert, das ausgefallene Triphenylphosphin abfiltriert und das Filtrat alkalisiert. Das gewünschte Produkt fällt dabei aus und wird nach Filtration und Trocknung in einer sehr hohen Reinheit und ausgezeichneter Ausbeute (95-98% der Theorie) gewonnen.

Als Lösungsmittel für die Umsetzung eignen sich organische Lösungsmittel, wie beispielsweise Tetrahydrofuran, Dimethoxyethan, Methanol, Ethanol oder Dimethylformamid. Als Basen werden organische Basen, wie Amine , z. B. Triethylamin, Ethyldiisopropylamin, oder anorganische Basen wie Kaliumcarbonat, Natriumcarbonat, Magnesiumcarbonat oder -hydroxyde verwendet. Die Reaktionszeit für die Umsetzung der Phthalidyl-3-triphenylphosphonium Salze beträgt 1 Stunde bei 40 ° C und für die Umsetzung mit Hydrazin 7 - 14 Stunden bei 50 - 70 **°** C.

Die als Edukte verwendeten Phthalidyl-3-triphenylphosphonium-Salze (Bromide und Chloride) sind nach literaturbekannten Methoden leicht zugänglich (J. Organometallic Chem. **1972**, 391; J. Org. Chem. **1973**, 4164).

Vorteile des erfindungsgemäßen Verfahrens gegenüber den aus dem Stand der Technik bekannten Verfahren sind die milderen Reaktionsbedingungen, deutlich bessere Ausbeute (> 90%) und insbesondere die Verwendung von stöchiometrischen Mengen Hydrazin. Die Reaktionen verlaufen vollständig und in einem geschlossenen System, so daß vor der Aufarbeitung kein Hydrazin im Reaktionsgemisch nachweisbar ist (Eintopfreaktion). Damit wird die Gefährdung durch diesen kanzerogen Stoff vermieden.

### Ausführungsbeispiele:

### Beispiel 1

### Herstellung von 4-(4-Pyridylmethyl)phthalazinon

500 g Phthalidyl-3-triphenylphosphonium-chlorid (1,160 mol) werden in 2250 ml Tetrahydrofuran (THF) suspendiert. Bei 5 ° C werden 110,7 ml Pyridin-4-aldehyd (124,2 g, 1,160 mol) hinzugegeben und anschließend werden 161,7 ml Triethylamin (117,4 g, 1,160 mol) zu der weißen Suspension dosiert. Nach beendeter Zugabe wird das Reaktionsgemisch 1 h bei 40 ° C gerührt, anschließend mit 62.0 ml Hydrazinhydrat (63,9 g, 1,276 mol) versetzt und 8 h bei 70 ° C gerührt. Danach werden 32,7 ml Essigsäureanhydrid (35,5 g, 0,348 mol) zugegeben und das Rühren für 2.5 h bei 20 ° C fortgesetzt. Anschließend wird das Reaktionsgemisch erst mit 1500 ml Wasser, dann mit 367 ml 4 M H₂SO₄-Lösung versetzt. Im Vakuum wird von dieser Reaktionslösung ca. 2500 ml THF/Wasser abdestilliert. Die erhaltene Suspension wird über eine Glasfritte filtriert. Das Filtrat mit 50%-iger NatriumhydroxidLösung bis auf pH 8.0 (ca. 185 ml) versetzt. Das ausgefallene Produkt wird abfiltriert, mit 450 ml Wasser gewaschen und bei 60°C getrocknet. Man erhält 264.2 g (96% der Theorie) eines leicht gelblichen Feststoffes.
Smp.: 193-194 °C. EI-MS (M+H⁾⁺ 242.

Analog zu diesem Beispiel erfolgt die Herstellung der anderen Derivate.

## Patentansprüche

1. Verfahren zur Herstellung von 4-(Heteroaryl-methyl)-1(2H)-phthalazinonen der allgemeinen Formel I 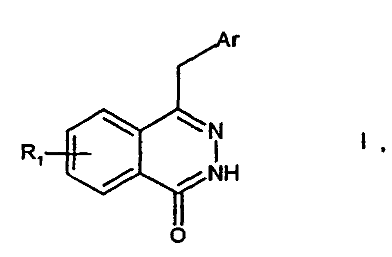 worin R¹ = Fluor, Chlor, Brom oder Wasserstoff und
Ar = Pyridin, Pyrazin oder Pyrimidin bedeutet, **dadurch gekennzeichnet, daß** substituierte Phthalidyl-3-triphenylphosphonium-Salze der allgemeinen Formel II 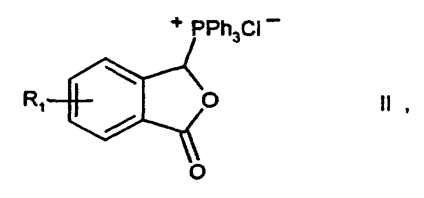 worin R¹ = Fluor, Chlor, Brom oder Wasserstoff bedeutet,
mit Aldehyden der allgemeinen Formel III
Ar-CHO III,
worin Ar = Pyridin, Pyrazin oder Pyrimidin bedeutet,
in Gegenwart einer Base und anschließender Reaktion mit Hydrazinhydrat, und gegebenenfalls unter sauren Bedingungen umgesetzt werden.

2. Verfahren nach Anspruch 1, wobei als Base Amine oder Alkali- oder Erdalkalihydroxide verwendet werden.

3. Verfahren nach Anspruch 1, wobei die Reaktion mit Hydrazinhydrat und anschließende Behandlung des Reaktionsgemisches mit Essigsäureanhydrid oder Essigsäure erfolgt.

4. Verfahren nach Anspruch 1, wobei die Reaktion mit 1-1.1 Eq. Hydrazinhydrat und anschließende Behandlung des Reaktionsgemisches mit 0,1-0,3 Eq. Essigsäureanhydrid erfolgt.

## Claims

1. Process for preparing 4-(heteroarylmethyl)-1(2H)-phthalazinones of the general formula I 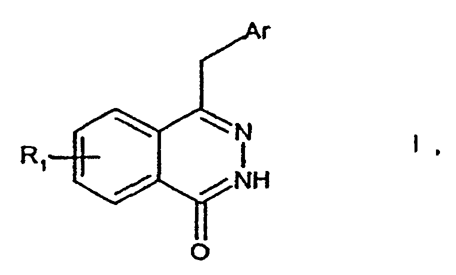 where R¹ = fluorine, chlorine, bromine or hydrogen and
Ar = pyridinyl, pyrazinyl or pyrimidinyl, **characterized in that** substituted phthalidyl-3-triphenylphosphonium salts of the general formula II 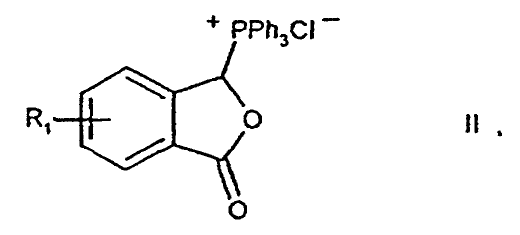 where R¹ = fluorine, chlorine, bromine or hydrogen are reacted with aldehydes of the general formula III
Ar-CHO III
where Ar = pyridinyl, pyrazinyl or pyrimidinyl in the presence of a base and subsequently reacted with hydrazine hydrate, optionally under acidic conditions.

2. Process according to Claim 1, in which the base used is an amine or an alkali metal hydroxide or alkaline earth metal hydroxide.

3. Process according to Claim 1, in which the reaction mixture is reacted with hydrazine hydrate and subsequently treated with acetic anhydride or acetic acid.

4. Process according to Claim 1, in which the reaction mixture is reacted with 1-1.1 eq. of hydrazine hydrate and subsequently treated with 0.1-0.3 eq. of acetic anhydride.

## Revendications

1. Procédé de préparation de 4-(hétéroarylméthyl)-1(2H)-phtalazinones de formule générale I 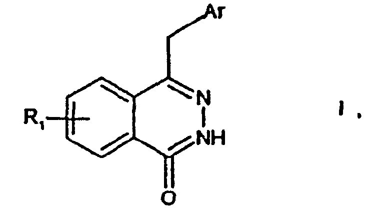 dans laquelle R₁ = fluor, chlore, brome ou hydrogène et Ar = pyridine, pyrazine ou pyrimidine, **caractérisé en ce qu'**on transforme des sels de phthalidyl-3-triphénylphosphonium substitués de formule générale II 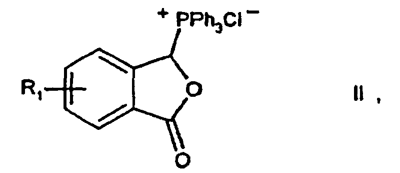 dans laquelle R₁ = fluor, chlore, brome ou hydrogène, avec des aldéhydes de formule générale III
Ar-CHO III,
dans laquelle Ar = pyridine, pyrazine ou pyrimidine, en présence d'une base et par réaction consécutive avec une hydrazine hydratée et le cas échéant dans des conditions acides.

2. Procédé selon la revendication 1, dans lequel on utilise comme base des amines ou des hydroxydes de métal alcalin ou alcalino-terreux.

3. Procédé selon la revendication 1, dans lequel la réaction est réalisée avec une hydrazine hydratée et par un traitement consécutif du mélange réactionnel avec de l'anhydride de l'acide acétique ou avec de l'acide acétique.

4. Procédé selon la revendication 1, dans lequel la réaction est réalisée avec 1 à 1,1 équivalent d'hydrazine hydratée et par un traitement consécutif du mélange réactionnel avec 0,1 à 0,3 équivalent d'anhydride de l'acide acétique.
